Europäisches Patentamt

(19) European Patent Office

Office européen des brevets

(11) Publication number: **0 245 784 B1**

# EUROPEAN PATENT SPECIFICATION

(45) Date of publication of patent specification: 24.07.91 (51) Int. Cl.⁵: **A61M 1/14**

(21) Application number: 87106646.0

(22) Date of filing: 07.05.87

(54) Oxygenator with microporous membrane.

(30) Priority: 12.05.86 IT 2039586

(43) Date of publication of application:
19.11.87 Bulletin 87/47

(45) Publication of the grant of the patent:
24.07.91 Bulletin 91/30

(84) Designated Contracting States:
DE FR GB IT

(56) References cited:
EP-A- 0 082 721
EP-A- 0 089 122
DE-A- 2 434 571

(73) Proprietor: **DIDECO S.p.A.**
**Via Galilei 3**
**I-41037 Mirandola (Province of Modena)(IT)**

(72) Inventor: **Fini, Massimo**
**Via Savonarola 3**
**I-41037 Mirandola (Provence of Modena)(IT)**
Inventor: **Vescovini, Pietro**
**Via degli Artigiani 26b**
**I-41036 Medolla (Provence of Modena)(IT)**

(74) Representative: **Modiano, Guido et al**
**MODIANO, JOSIF, PISANTY & STAUB**
**Modiano & Associati Via Meravigli, 16**
**I-20123 Milano(IT)**

Rank Xerox (UK) Business Services

## Description

The present invention relates to a microporous membrane oxygenator according to the preamble of claim 1.

It is known that in the course of some surgical operations an extracorporeal circulation of the blood is necessary, during which it is necessary to supply the blood with oxygen, and for this purpose said blood is made to pass through devices, known as oxygenators, which satisfy this need.

A very common type of oxygenator is provided internally with a microporous membrane separating a blood compartment and an oxygen compartment and through which oxygen passes into the blood by diffusion, this membrane can have different embodiments, there being either plane membrane oxygenators or oxygenators in which the membrane has the form of a bundle of capillaries.

In order to have a correct diffusion of the oxygen into the blood of the patient, without causing the danger of the formation of bubbles in the blood, it is necessary that the pressures of the two fluids be in a ratio comprised within an optimum value range which is normally ensured by the structural and functional characteristics of the circuits of said fluids, but it can occur that accidental causes, such as for example a wrong maneuver on the part of the operators, causes an increase in the pressure of the oxygen to values dangerous for the safety of the patient.

DE-A-2434571 shows a blood oxygenator comprising a safety valve for venting the oxygen when its pressure becomes excessive. The safety valve incorporates a porous membrane which permits the outflow of gases but not of liquids.

The aim of the present invention is to provide a microporous membrane oxygenator wherein the limiting to correct values of oxygen pressure is ensured, and this is achieved in a very simple manner, so as to ensure the maximum reliability in operation.

The aim proposed is achieved by a microporous membrane oxygenator, according to appended claim 1.

Further characteristics and advantages of the invention will become apparent from the description of some preferred, but not exclusive, embodiments of the invention, illustrated only by way of non-limitative example in the accompanying drawings, where both fluids, that is blood and oxygen, circulating in the apparatus are absent, and where:

figure 1 is a longitudinal cross section view of a capillary oxygenator provided with the valve according to a first aspect of the invention;

figure 2 is a detailed longitudinal cross section view of the insertion into the oxygenator of a valve according to the first aspect, but positioned differently from the preceding one;

figure 3 is a detailed longitudinal cross section view of a valve according to a second aspect of the invention;

figure 4 is a detailed longitudinal cross section view of a valve according to a third aspect of the invention;

Figure 5 is a detailed transversal cross section view of a valve according to a fourth aspect of the invention; and

Figure 6 is a longitudinal cross section view of the elastic membrane of the valve of Figure 5 along the line VI-VI;

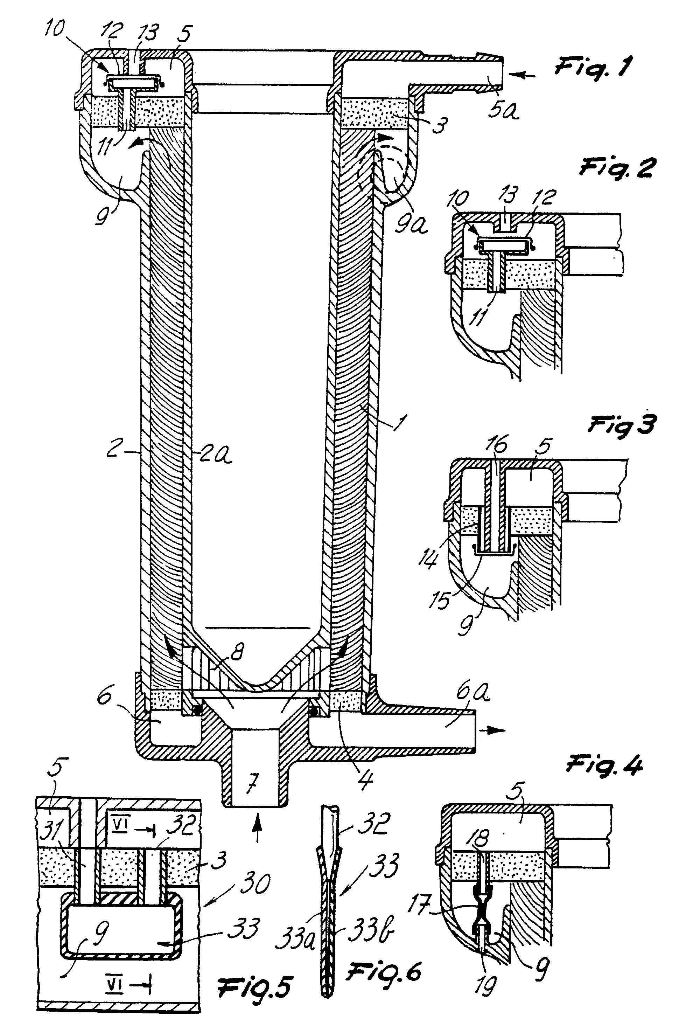

With reference to figure 1, the reference numeral 1 generally indicates a bundle of capillaries made in microporous membrane and contained inside the walls 2 and 2a of the cylindrical structure of the oxygenator and sunk at its ends in the polyurethanic resin fillers 3 and 4, known as "potting"; said capillaries are adapted for conveying oxygen from the upper annular compartment 5 with inlet connection 5a to the lower annular compartment 6 with outlet connection 6a.

In counterflow with respect to the oxygen, the capillaries of the bundle 1 are externally in contact with the blood, which receives oxygen by diffusion through the walls, made of microporous membrane, of said capillaries. The blood enters the apparatus from the lower connection 7, passes through the window-fitted portion 8, travels along the space comprised between the cylindrical walls 2 and 2a making contact with the capillaries, flows into the annular compartment 9 and eventually exits through the connection 9a, as shown by the arrows of the figure.

The safety valve according to a first aspect of the invention is generally indicated by the reference numeral 10: it comprises the conduit 11 inserted in the potting 3 and upwardly widened. The conduit 11 is open at the lower end onto the blood compartment 9 and occluded at the upper end by the planar elastic membrane 12, made for example of rubber, which is arranged with its central portion facing the end of the conduit 13 leading into the atmosphere and in contact therewith when at rest, as illustrated in the figure; in this manner the elastic membrane 12 is on one side in contact with the blood and one the other side in contact with the oxygen contained in the compartment 5.

With the valve 10 arranged as illustrated in figure 1, with the membrane 12 in contact, at rest, with the end of the conduit 13, the oxygen flows into the atmosphere only when its pressure exceeds the pressure of the blood by such an amount as to deform the elastic membrane 12, thus lowering it. The amount can be determined with a proper selection of the thickness and of the elastic characteristics of the material of which the membrane is made, so as to be certain that no damage is caused to the patient by the forming of bubbles in the blood.

The discharge of the oxygen into the atmosphere obviously lasts until the oxygen pressure is returned to correct values.

Different functional conditions are determined with a safety valve 10 identical to the one illustrated in figure 1, but positioned, as illustrated in figure 2, with the elastic membrane 12 detached, when at rest, from the end of the conduit 13.

In this case, the oxygen flows into the atmosphere not only when its pressure is greater than the pressure of the blood, but also, reversing the situation, when the pressure of the blood is greater than the pressure of the oxygen by a quantity smaller than the quantity determining an upward deformation of the membrane 12, such as to make it occlude the end of the conduit 13.

Thus, as can be seen, by suitably varying the position of the valve 10 and the constructive characteristics thereof, it is possible to obtain a very wide range of different functional conditions, capable of satisfying any requirement.

Figure 3 illustrates a second aspect of the safety valve according to the invention; said valve comprises a first conduit 14 in communication at one end with the oxygen compartment 5 and occluded by the plane elastic membrane 15 at the other end in communication with the blood compartment 9. A second coaxial conduit 16 internal to the first conduit 14 has an end facing the membrane 15 and the other end open onto the atmosphere.

By varying the distance of the end of the conduit 16 from the elastic membrane 15 and the constructive characteristics thereof, it is possible to obtain a range of functional performance of the type described with reference to the first aspect of the invention.

A third aspect of the valve according to the invention is illustrated in figure 4, comprising a tubular elastic membrane 17 externally in contact with the blood compartment 9 and adapted to connect the facing ends of the conduit 18 open onto the oxygen compartment 5, and of the conduit 19 open onto the atmosphere.

If the tubular membrane has, as in the example in the figure, an obstructed section when at rest, the discharge of the oxygen into the atmosphere will occur only when the pressure of the oxygen exceeds the pressure of the blood by a certain value; conversely, if the tubular membrane at rest has an unobstructed section, the discharge of the oxygen occurs until the pressure of the blood does not exceed, by a certain value sufficient to make the walls of the membrane collapse and close onto one another, the pressure of the oxygen.

Figures 5 and 6 illustrate a valve according to a fourth aspect of the invention.

The valve 30 comprises a substantially rectangular bag-like elastic membrane 33 which is located within the blood compartment 9. The valve 30 further comprises a first conduit 31 communicating, at one end, with the atmosphere and, at the other end, with the interior of the membrane 33 and a second conduit 32 communicating, at one end, with the oxygen compartment 5 and, at the other end, with the interior of the membrane 33.

The membrane 33 is preferably obtained by welding together two sheets 33a and 33b of elastic material (e.g. PVC ) along their edges and including the ends of the conduits 31 and 32.

When at rest the sheets 33a and 33b are collapsed on each other thus blocking the communication between the conduits 31 and 32.

If the oxygen pressure exceeds a certain value, relatively to the blood pressure, the membrane 33 would "inflate" thus connecting the two conduits 31 and 32 so that the oxygen would discharge into the atmosphere until the proper pressure is restored.

The invention described is susceptible to numerous modifications and variations, besides the ones described, also relatively to plane microporous membrane, oxygenators, all of which are within the scope of the inventive concept as claimed.

In the practical embodiment of the invention, all the details may be replaced with other technically equivalent elements: moreover, the materials employed, as well as the shapes and dimensions, may be any.

## Claims

1. Microporous membrane oxygenator, comprising an oxygen compartment and a blood compartment separated by a microporous membrane through which oxygen passes into the blood by diffusion and a safety valve (10,30) for connecting the oxygen compartment to the atmosphere upon an increase of oxygen pressure above a selected value, characterized in that the opening and closing of the safety valve (10,30) are controlled by at least one elastic membrane (12,15,17,33) having at least one side in communication with said oxygen compartment and at least one other side in communication with said blood compartment, said at least one elastic membrane being impervious to oxygen and blood.

2. Oxygenator according to claim 1, characterized in that the safety valve (10) comprises a first conduit (11) in communication at one end thereof with said blood compartment (9) and occluded at the other end by a planar elastic membrane (12) which is arranged with a portion thereof facing the inlet of a second conduit (13) in communication at one end thereof with said oxygen compartment (5) and in communication at the other end thereof with the atmosphere.

3. Oxygenator according to claims 1 and 2, characterized in that said elastic membrane (12) facing with a portion thereof said inlet of a conduit (13) in communication with the atmosphere is in contact with said inlet.

4. Oxygenator according to claims 1 and 2, characterized in that said elastic membrane (12) facing with a portion thereof said inlet of a conduit (13) in communication with the atmosphere is spaced apart from said inlet.

5. Oxygenator according to claim 1, characterized in that the safety valve (10) comprises a first conduit (14) in communication at one end with the oxygen compartment (5) and occluded, at the other end comprised within the blood compartment (9), by a planar elastic membrane (15), and further comprising a second conduit (16), coaxial and internal to said first conduit (14), and having an end facing said elastic membrane (15) and the other end in communication with the atmosphere.

6. Oxygenator according to claims 1 and 5, characterized in that said end of said second conduit (16) facing said elastic membrane (15) is in contact therewith.

7. Oxygenator according to claims 1 and 5, characterized in that said end of said second conduit (16) facing said elastic membrane (15) is spaced apart therefrom.

8. Oxygenator according to claim 1, characterized in that said safety valve (10) comprises a tubular elastic membrane (17) externally in contact with said blood compartment (9), adapted to connect the facing ends of two conduits (18,19) open at their other ends respectively one (18) in communication with said oxygen compartment (5) and the other one (19) in communication with the atmosphere.

9. Oxygenator according to claims 1 and 8, characterized in that said tubular elastic membrane (17) is configured, when at rest, so that its walls collapse and contact one another to obstruct oxygen flow between said oxygen compartment (5) and the atmosphere.

10. Oxygenator according to claim 8, characterized in that said tubular elastic membrane (17) is configured, when at rest, such that there is an unobstructed oxygen flow between said oxygen compartment (5) and the atmosphere.

11. Oxygenator, according to claim 1, characterized in that said safety valve (30) comprises a substantially rectangular bag-like elastic membrane (33), inserted into the blood compartment(9) and having an interior, delimited by inner faces (33a,33b), communicating with the ends of first (31) and second (33) conduits, said first and second conduits (31,33) communicating at their other ends with the atmosphere and the oxygen compartment (5) respectively, said bag-like membrane (33) being elastically deformable such that said inner faces (33a,33b) of said bag-like membrane (33) can be moved apart to permit passage of oxygen therebetween, upon an increase of oxygen pressure above a selected value.

12. Oxygenator, according to claims 1 and 11, characterized in that said bag-like elastic membrane (33) comprises at least two sheets (33a,33b) of elastic material welded at their perimeter with the inclusion of said ends of said conduits (31,32).

**Revendications**

1. Oxygénateur à membrane microporeuse, comprenant un compartiment d'oxygène et un compartiment de sang séparés par une membrane microporeuse à travers laquelle l'oxygène passe dans le sang par diffusion, et une soupape de sécurité (10,30) pour relier le compartiment d'oxygène à l'atmosphère lors d'une augmentation de la pression d'oxygène au-dessus d'une valeur choisie,
caractérisé en ce que l'ouverture et la fermeture de la soupape de sécurité (10,30) sont contrôlées par au moins une membrane élastique (12,15,17,33) ayant au moins un côté en communication avec ledit compartiment d'oxygène et au moins un autre côté en communication avec ledit compartiment de sang, ladite membrane élastique étant imperméable à l'oxygène et au sang.

2. Oxygénateur selon la revendication 1,
caractérisé en ce que la soupape de sécurité (10) comprend un premier conduit (11) en communication à une extrémité de celui-ci avec ledit compartiment de sang (9) et fermé à l'autre extrémité par une membrane élastique plane (12) qui est agencée avec une partie de celle-ci faisant face à l'entrée d'un second conduit (13) en communication à une extrémité de celui-ci avec ledit compartiment d'oxygène (5) et en communication a l'autre extrémité de celui-ci avec l'atmosphère.

3. Oxygénateur selon les revendications 1 et 2, caractérisé en ce que ladite membrane élastique (12) dont une partie fait face à ladite entrée d'un conduit (13) en communication avec l'atmosphère est en contact avec ladite entrée.

4. Oxygénateur selon les revendications 1 et 2, caractérisé en ce que ladite membrane élastique (12) dont une partie fait face à ladite entrée d'un conduit (13) en communication avec l'atmosphère est espacée de ladite entrée.

5. Oxygénateur selon la revendication 1, caractérisé en ce que la soupape de sécurité (10) comprend un premier conduit (14) en communication à une extrémité avec le compartiment d'oxygène (5) et fermé, à l'autre extrémité comprise dans le compartiment de sang (9), par une membrane élastique plane (15), et comprenant de plus un second conduit (16), coaxial et interne audit premier conduit (14), et ayant une extrémité faisant face à ladite membrane élastique (15) et l'autre extrémité en communication avec l'atmosphère.

6. Oxygénateur selon les revendications 1 et 5, caractérisé en ce que ladite extrémité dudit second conduit (16) faisant face à ladite membrane élastique (15) est en contact avec celle-ci.

7. Oxygénateur selon les revendications 1 et 5, caractérisé en ce que ladite extrémité dudit second conduit (16) faisant face à ladite membrane élastique (15) est espacée de celle-ci.

8. Oxygénateur selon la revendication 1, caractérisé en ce que ladite soupape de sécurité (10) comprend une membrane élastique tubulaire (17) extérieurement en contact avec ledit compartiment de sang (9), adaptée pour relier les extrémités en vis-à-vis de deux ccnduits (18,19) ouverts à leurs autres extrémités, respectivement l'un (18) en communication avec ledit compartiment d'oxygène (5) et l'autre (19) en communication avec l'atmosphère.

9. Oxygénateur selon les revendications 1 et 8, caractérisé en ce que ladite membrane élastique tubulaire (17) est configurée, au repos, de sorte que ses parois s'affaissent et sont au contact l'une de l'autre pour arrêter le flux d'oxygène entre ledit compartiment d'oxygène (5) et l'atmosphère.

10. Oxygénateur selon la revendication 8, caractérisé en ce que ladite membrane élastique tubulaire (17) est configurée, au repos, de sorte qu'il existe un flux d'oxygène non empêché entre ledit compartiment d'oxygène (5) et l'atmosphère.

11. Oxygénateur selon la revendication 1, caractérisé en ce que ladite de soupape de sécurité (30) comprend une membrane élastique en forme de sac sensiblement rectangulaire (33), insérée dans le compartiment de sang (9) et ayant une partie interne, délimitée par des faces internes (33a,33b), communiquant avec les extrémités de premier (31) et second (32) conduits, lesdits premier et second conduits (31,32) communiquant à leurs autres extrémités avec l'atmosphère et le compartiment d'oxygène (5), respectivement, ladite membrane en forme de sac (33) étant élastiquement déformable de sorte que lesdites faces internes (33a,33b) de ladite membrane en forme de sac (33) peuvent être éloignées pour permettre le passage de l'oxygène entre elles, lorsque la pression d'oxygène augmente au-dessus d'une valeur choisie.

12. Oxygénateur selon les revendications 1 et 11, caractérisé en ce que ladite membrane élastique en forme de sac (33) comprend au moins deux feuilles (33a,33b) de matière élastique soudées sur leur périmètre avec l'inclusion desdites extrémités desdits conduits (31,32).

**Patentansprüche**

1. Oxygenerator mit mikroporöser Membrane, enthaltend

ein Oxygenabteil und ein Blutabteil, die durch eine mikroporöse Membrane voneinander getrennt sind, wobei über die mikroporöse Membrane Oxygen in das Blut diffundiert, sowie

ein Sicherheitsventil (10, 30) zur Verbindung des Oxygenabteils mit der Atmosphäre bei einem Ansteigen von Oxygendruck über einen gewählten Wert,

dadurch gekennzeichnet, daß das Öffnen und Schließen des Sicherheitsventils (10, 30) durch wenigstens eine elastische Membrane (12, 15, 17, 33) gesteuert wird, die auf wenigstens einer Seite mit dem Oxygenabteil und mit wenigstens einer anderen Seite in Kontakt mit dem Blutabteil steht, wobei diese Membrane für Oxygen und Blut undurchlässig ist.

2. Oxygenerator nach Anspruch 1, dadurch gekennzeichnet, daß das Sicherheitsventil (10) eine erste Leitung (11) enthält, die an einem Ende mit dem Blutabteil (9) in Verbindung steht und am anderen Ende durch eine planare, elastische Membrane (12) abgeschlossen ist, die so angeordnet ist, daß ein Teil dem Einlaß einer zweiten Leitung (13) gegenübersteht, die über ein Ende mit dem Oxygenabteil (5) und über das andere Ende mit der Atmosphäre in Verbindung steht.

3. Oxygenerator nach den Ansprüchen 1 und 2, dadurch gekennzeichnet, daß die elastische Membrane (12), die zum Teil dem Einlaß einer mit der Atmosphäre verbundenen Leitung (13) gegenübersteht, diesen Einlaß berührt.

4. Oxygenerator nach den Ansprüchen 1 und 2, dadurch gekennzeichnet, daß die elastische Membrane (12), die zum Teil dem Einlaß einer mit der Atmosphäre verbundenen Leitung (13) gegenübersteht, mit Abstand zu diesem Einlaß angeordnet ist.

5. Oxygenerator nach Anspruch 1, dadurch gekennzeichnet, daß das Sicherheitsventil (12) eine erste Leitung (14) enthält, die an einem Ende mit dem Oxygenabteil (5) in Verbindung steht und am anderen, im Blutabteil (9) aufgenommenen Ende durch eine planare, elastische Membrane (15) verschlossen ist, und ferner eine zweite Leitung (16) vorgesehen ist, die koaxial zu und innerhalb der ersten Leitung (14) verlaufend angeordnet ist, wobei die zweite Leitung (16) ein Ende aufweist, das der elastischen Membrane (15) gegenübersteht, und über das andere Ende mit der Atmosphäre in Verbindung steht.

6. Oxygenerator nach den Ansprüchen 1 und 5, dadurch gekennzeichnet, daß das Ende der zweiten Leitung (16), das der elastischen Membrane (15) gegenübersteht, diese berührt.

7. Oxygenerator nach den Ansprüchen 1 und 5, dadurch gekennzeichnet, daß das Ende der zweiten Leitung (16), das der elastischen Membrane (15) gegenübersteht, zu dieser mit Abstand angeordnet ist.

8. Oxygenerator nach Anspruch 1, dadurch gekennzeichnet, daß das Sicherheitsventil (10) eine rohrartige, elastische Membrane (17) enthält, die nach außen in Verbindung mit dem Blutabteil (9) steht, und die einander gegenüberstehenden Enden von zwei an ihren anderen Enden jeweils offenen Leitungen (18, 19) verbindet, wobei die eine (18) mit dem Oxygenabteil (5) und die andere (19) mit der Atmosphäre verbunden ist.

9. Oxygenerator nach den Ansprüchen 1 und 8, dadurch gekennzeichnet, daß die rohrartige, elastische Membran (17) so geformt ist, daß ihre Wände kollodieren und sich gegenseitig berühren, wenn sie sich in Ruhelage befinden, so daß eine Oxygenströmung zwischen dem Oxygenabteil (5) und der Atmosphäre verhindert wird.

10. Oxygenerator nach Anspruch 8, dadurch gekennzeichnet, daß die rohrartige, elastische Membrane (17) so geformt ist, daß eine ungehinderte Oxygenströmung zwischen dem Oxygenabteil (5) und der Atmosphäre stattfindet, wenn sich die rohrartige elastische Membrane (17) in Ruhelage befindet.

11. Oxygenerator nach Anspruch 1, dadurch gekennzeichnet, daß das Sicherheitsventil (30) eine im wesentlichen rechteckige, sackartige elastische Membrane (33) aufweist, die in das Blutabteil (9) eingeführt wird und ein Inneres aufweist, das durch innere Seiten (33a, 33b) begrenzt mit den Enden einer ersten (31) und einer zweiten (32) Leitung verbunden ist, wobei die erste und die zweite Leitung (31, 32) über ihr jeweils anderes Ende mit der Atmosphäre bzw. dem Oxygenabteil (5) in Verbindung stehen, und wobei die sackartige Membrane (33) elastisch verformbar ist, so daß die inneren Seiten (33a, 33b) dieser sackartigen Membrane (33) entfernt werden können, um dazwischen einen Oxygenfluß zu ermöglichen, sofern der Oxygendruck über einen bestimmten Wert ansteigt.

12. Oxygenerator nach den Ansprüchen 1 und 11, dadurch gekennzeichnet, daß die sackartige elastische Membrane (33) wenigstens zwei Flächen (33a, 33b) aus elastischem Material enthält, die an ihrem Umfang unter Einschluß der Enden der Leitungen (31, 32) verschweißt sind.

*Fig. 1*

*Fig. 2*

*Fig 3*

*Fig. 4*

*Fig.5*

*Fig.6*